# EUROPÄISCHE PATENTSCHRIFT

(11) **EP 3 403 476 B1**
(45) Veröffentlichungstag und Bekanntmachung des Hinweises auf die Patenterteilung: **28.07.2021**
(21) Anmeldenummer: 16819283.9
(22) Anmeldetag: 09.12.2016
(51) Int. Cl.: H05K 7/14, H05K 7/16

(54) **SCHALTSCHRANK FÜR ABGASMESSANLAGEN**
SWITCHGEAR CABINET FOR EXHAUST GAS MEASURING FACILITIES
ARMOIRE DE COMMANDE POUR SYSTÈMES DE MESURE DE GAZ D'ÉCHAPPEMENT

(30) Priorität: 15.01.2016 DE 102016100634
(43) Veröffentlichungstag der Anmeldung: 21.11.2018
(73) Patentinhaber: AVL Emission Test Systems GmbH, 41460 Neuss (DE)
(72) Erfinder: DICKOW, Achim, 42555 Velbert (DE)
(74) Vertreter: terpatent Patentanwälte ter Smitten Eberlein-Van Hoof Rütten Daubert
(86) Internationale Anmeldenummer: PCT/EP2016/080417
(87) Internationale Veröffentlichungsnummer: WO 2017/121553

(56) Entgegenhaltungen:
- EP-A1- 2 317 833
- AT-U1- 2 350
- US-A- 3 755 716
- US-A1- 2004 029 617
- US-A1- 2008 040 885
- US-A1- 2012 119 632

## Beschreibung

Die Erfindung betrifft einen Schaltschrank für Abgasmessanlagen mit einem ersten Schrankkörper, in dem erste Messgeräte zur Analyse von Probengasen angeordnet sind, einer Tür zum Öffnen und Schließen einer offenen Frontseite des ersten Schrankkörpers, die drehbar am ersten Schrankkörper befestigt ist und einer Bedieneinheit, die an der Tür angeordnet ist.

Derartige Schaltschränke werden beispielsweise an Rollenprüfständen von Kraftfahrzeugen verwendet. Diese Schaltschränke beinhalten einerseits die Ansteuerung und Elektronik der in der Abgasmessanlage, welche beispielsweise als CVS-Anlage (constant volume sample) ausgeführt sein kann, vorhandenen Regelglieder und andererseits die zur Analyse des Abgases notwendigen Messgeräte zur Bestimmung der Schadstoffmengen im Abgas, wie beispielsweise Flammenionisationsdetektoranalysegeräte zur Bestimmung von Kohlenwasserstoffen oder über einen Gaschromatographen zur Bestimmung von Methanmengen, Chemilumineszenzdetektoranalysegeräte zur Bestimmung von Stickoxidmengen im Abgas, Infrarotdetektoranalysegeräte zur Bestimmung verschiedener aktiver Komponenten wie Kohlenmonoxid, Kohlendioxid oder Kohlenwasserstoffverbindungen im Abgas. Entsprechend ist der Schaltschrank über Leitungen, welche über entsprechende Kupplungen an den Schaltschrank angeschlossen werden, mit den Probenahmesonden der Abgasmessanlage verbunden.

Jeder Schaltschrank trägt entsprechend die Messgeräte und Steuerungen für eine Messlinie und weist an seiner an der Frontseite angeordneten Tür eine Öffnung auf, in der sich die Bedieneinheit befinden kann und über die die verschiedenen Messgeräte bedient werden können, so dass die Analysedaten unmittelbar zur Verfügung stehen.

Aus der EP 2 317 833 A1 ist beispielsweise ein zweiteiliger Schaltschrank bekannt, bei de min einem ersten Schrankköper, der durch eine Tür verschließbar ist, die Messgeräte der Abgasanalyseeinheit angeordnet sind. In einem zweiten Schrankkörper, der hinter dem ersten Schrankkörper platzierbar ist, befinden sich die Gasanschlüsse sowie Ventile und Pumpen der Anlage. Problematisch ist, dass der ersten Schrankkörper zum zweiten frei bewegbar ist.

AT 002 350 U1 offenbart einen Schrank für Messgeräte, der aus zwei durch ein Scharnier verbundenen Teilen besteht.

Des Weiteren ist aus der US 3,755,716 ein Regalsystem für eine Telefonanlage bekannt, bei dem in einem verschließbaren Schrankkörper mehrere drehbare Regale aufgehängt sind.

In den meisten Einrichtungen zur Abgasanalyse sind jedoch mehrere Rollenprüfstände, Motorprüfstände beziehungsweise Abgasmesslinien untergebracht, an denen zur Zeitersparnis simultan Messungen an verschiedenen Fahrzeugen oder Motoren durchgeführt werden. Das Bedienpersonal muss entsprechend entweder zwischen den Schaltschränken wechseln oder es ist Bedienpersonal für jede einzelne Anlage vorzusehen. Zusätzlich werden durch die gehobenen Abgasnormen immer neue Messungen notwendig, so dass zusätzlich Raum benötigt wird, um die entsprechenden Geräte unterzubringen, so dass Platzprobleme in den dafür vorhandenen Hallen entstehen.

Es stellt sich daher die Aufgabe, einen Schaltschrank für Abgasmessanlagen zur Verfügung zu stellen, mit dem zumindest zwei Abgasmesslinien simultan über eine Bedieneinheit bedient werden können oder zusätzliche Messgeräte im Schrank platziert werden können, deren Erreichbarkeit dennoch erhalten bleiben soll. Der verwendete Bauraum soll trotz einer hohen Anzahl an zu verwendenden Messgeräten möglichst wenig Raum einnehmen. Dennoch sollen alle Instrumente und Anschlüsse gut zugänglich sein. Auch sollen die Kosten eines derartigen Schaltschranks im Vergleich zur bekannten Lösung reduziert werden.

Diese Aufgabe wird durch einen Schaltschrank für Abgasmessanlagen mit den Merkmalen des Hauptanspruchs 1 gelöst.

Dadurch, dass der erste Schrankkörper relativ zu einem zweiten Schrankkörper geführt bewegbar ist, in dem zweite Messgeräte angeordnet sind und der eine zweite offene Frontseite aufweist, welche durch die geführte Relativbewegung des ersten Schrankkörpers zum zweiten Schrankkörper verschließbar und freigebbar ist, wird eine kompakte platzsparende Einheit geschaffen, bei der eine gute Zugänglichkeit beider Schränke sichergestellt wird. Durch die kompakte Einheit ist es möglich, auch mehrere Messlinien über eine Bedieneinheit anzusteuern. Da auf eine Schrankwand an der Frontseite verzichtet werden kann, werden die Kosten reduziert.

Vorzugsweise ist der zweite Schrankkörper über ein Gelenk am ersten Schrankkörper befestigt und der erste Schrankkörper ist zum zweiten Schrankkörper um das Gelenk drehbar angeordnet. Entsprechend können die Schaltschränke zueinander aufgeklappt werden, so dass eine gute Erreichbarkeit aller im Innern beider Schränke angeordneter Teile erzielt wird.

In einer besonders bevorzugten Ausführungsform sind die Messgeräte im zweiten Schrankkörper elektrisch mit der Bedieneinheit an der Tür des ersten Schrankkörpers verbunden. Dies reduziert die Kosten erheblich, da eine Bedieneinheit für den zweiten Schaltschrank eingespart werden kann. Zusätzlich wird die simultane Bedienung zweier Abgasmesslinien über nur eine Bedieneinheit bei weiterhin guter Zugänglichkeit ermöglicht.

Besonders vorteilhaft ist es entsprechend, wenn die Messgeräte im ersten Schrankkörper einer ersten Abgasmesslinie zugeordnet sind und die Messgeräte im zweiten Schrankkörper einer zweiten Abgasmessanlage zugeordnet sind. Somit können zwei identische Anlagen simultan betrieben und über den einen Doppelschaltschrank gesteuert werden. Bei Fehlern wird eine Zuordnung zu den entsprechenden Messgeräten ebenso wie eine korrekte Montage erleichtert.

In einer bevorzugten Ausbildung der Erfindung sind an einem Boden des ersten Schrankkörpers Rollen angeordnet. Durch diese Rollen lässt sich der Schrankkörper ohne hohen Kraftaufwand relativ zum zweiten Schrankkörper aufklappen, so dass die Erreichbarkeit des zweiten Schrankkörpers verbessert wird.

Insbesondere ist es vorteilhaft, wenn der erste Schrankkörper mit seiner Rückwand gegen die offene Frontseite des zweiten Schrankkörper anliegt. Bei einer derartigen Anordnung können zwei identische Schrankkörper verwendet werden, wodurch aufgrund der höheren Anzahl zu verwendender Gleichteile erneut Kosten reduziert werden.

Eine bevorzugte Ausführung ergibt sich daher dadurch, dass der erste Schrankkörper und der zweite Schrankkörper sowie die im ersten Schrankkörper und im zweiten Schrankkörper angeordneten Messgeräte gleich sind. So wird die Anzahl verwendeter Gleichteile maximiert, was erneut die Kosten reduziert und die Montage erleichtert.

Ein besonders einfaches und geführtes Aufklappen zur verbesserten Öffnung des Schrankes insbesondere um 180° zur verbesserten Erreichbarkeit ergibt sich, wenn der erste Schrankkörper über mehrere an einer ersten Seitenwand angeordnete Scharniere an einer ersten Seitenwand des zweiten Schrankkörpers drehbar befestigt ist und an den jeweils gegenüberliegenden zweiten Seitenwänden der beiden Schrankkörper Verschlussmittel angeordnet sind, über die die zweite Seitenwand des ersten Schrankkörpers mit der zweiten Seitenwand des zweiten Schrankkörpers verbindbar ist. So wird eine geführte vollständige Öffnung des zweiten Schrankkörpers ermöglicht.

Vorzugsweise weist die Bedieneinheit einen Monitor, insbesondere einen Touchscreen auf, der in einer Öffnung der Tür angeordnet ist. Dies ermöglicht eine geführte einfache Bedienbarkeit und erhöht die Übersichtlichkeit der erzeugten Messdaten.

Es wird somit ein Schaltschrank für Abgasmessanlagen geschaffen, bei dem mehrere Abgasmesslinien parallel und simultan gefahren werden können, deren Steuerung lediglich über den einen Schaltschrank über eine Bedieneinheit von einer Bedienperson durchgeführt werden kann. Der verwendete Bauraum sowie die Kosten zum Aufbau und für die Montage werden im Vergleich zu bekannten Ausführungsformen deutlich reduziert. Dennoch bleibt eine sehr gute Erreichbarkeit aller verwendeten Messgeräte und Anschlüsse erhalten.

Ein Ausführungsbeispiel eines erfindungsgemäßen Schaltschranks für eine Abgasmessanlage ist in den Figuren dargestellt und wird nachfolgend beschrieben.

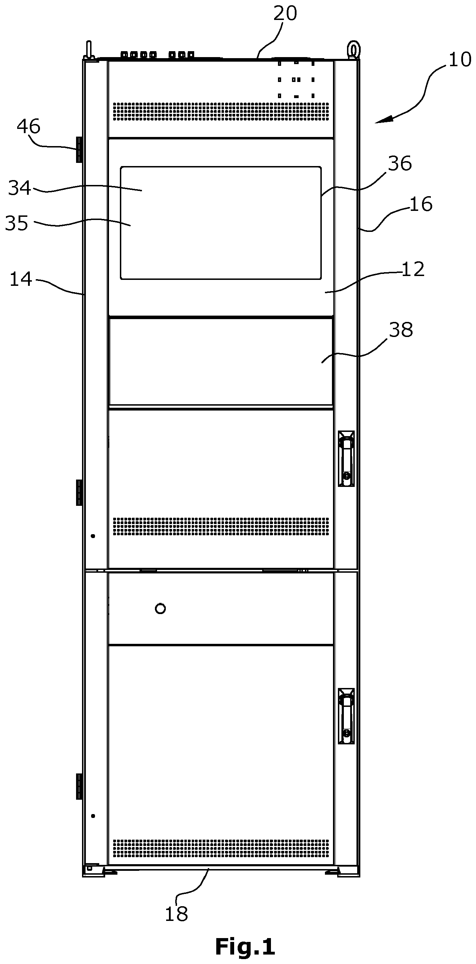

Figur 1 zeigt eine Frontansicht eines erfindungsgemäßen Schaltschranks für eine Abgasmessanlage im geschlossenen Zustand eines ersten Schrankkörpers.

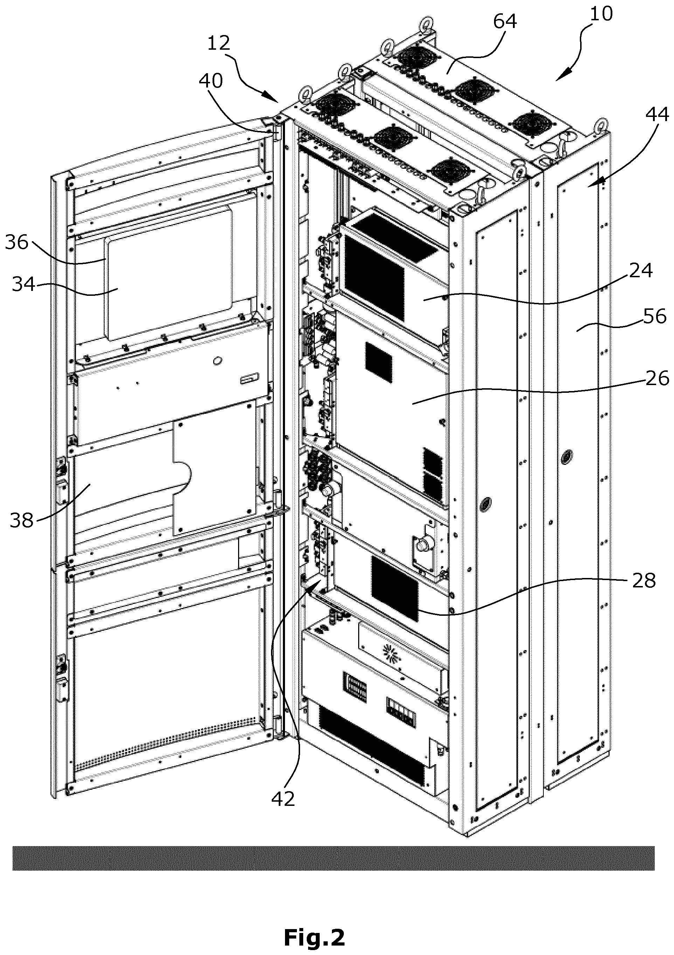

Figur 2 zeigt eine perspektivische Ansicht des erfindungsgemäßen Schaltschranks aus Figur 1 in geöffnetem Zustand des ersten Schrankkörpers.

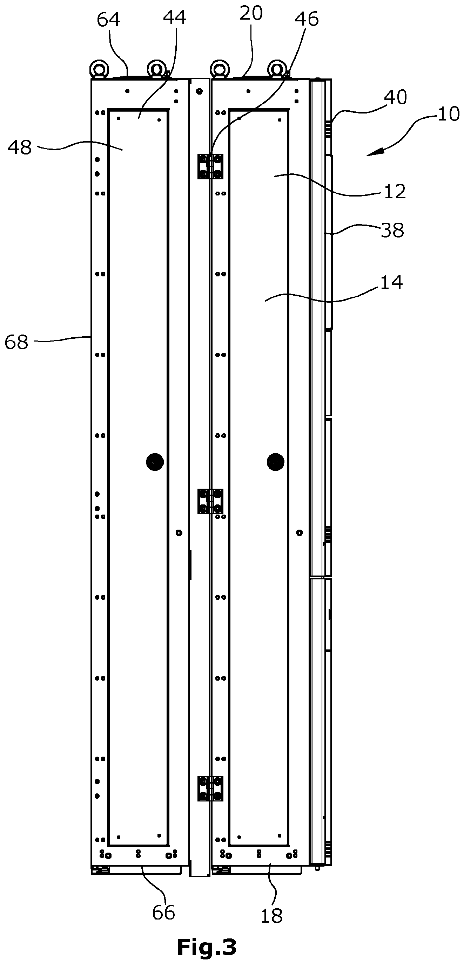

Figur 3 zeigt eine Seitenansicht des erfindungsgemäßen Schaltschranks im geschlossenen Zustand.

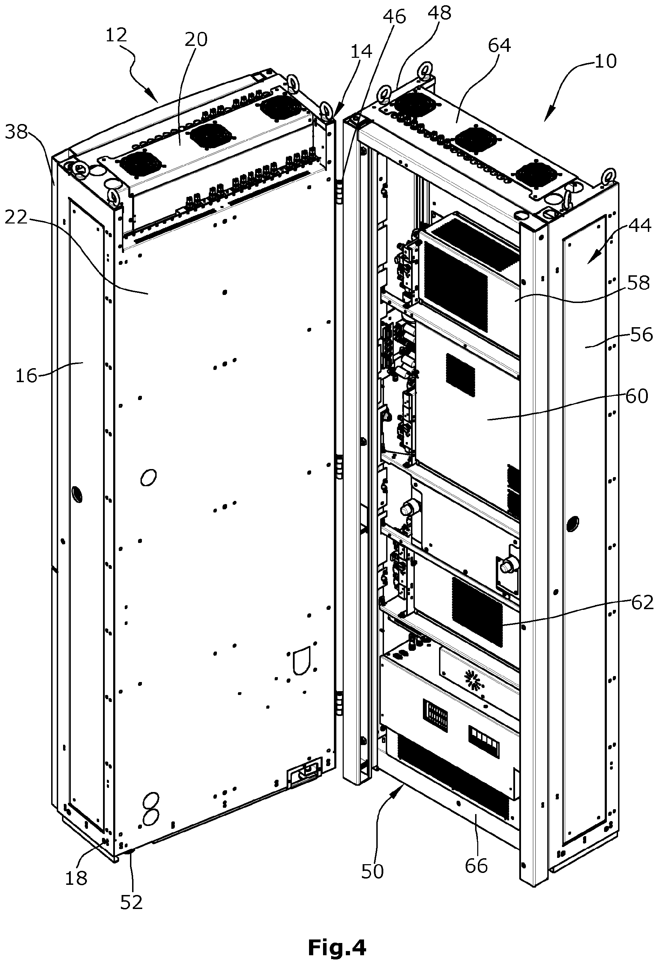

Figur 4 zeigt eine perspektivische Seitenansicht des erfindungsgemäßen Schaltschranks im geöffneten Zustand eines zweiten Schrankkörpers.

Der erfindungsgemäße Schaltschrank 10 für eine Abgasmessanlage besteht aus einem ersten Schrankkörper 12 mit einer ersten Seitenwand 14, einer gegenüberliegenden Seitenwand 16, einem Boden 18 und einer Decke 20 sowie einer Rückwand 22. Im Innern dieses Schrankkörpers 12 sind ein Flammenionisationsdetektoranalysegerät 24 zur Bestimmung von Kohlenwasserstoffen, ein Chemilumineszenzdetektoranalysegerät 26 zur Bestimmung von Stickoxidmengen im Abgas sowie ein Infrarotdetektoranalysegerät 28 zur Bestimmung verschiedener aktiver Komponenten wie Kohlenmonoxid, Kohlendioxid oder Kohlenwasserstoffverbindungen befestigt. Diese Messgeräte 24, 26, 28 sind über Leitungen mit Abgasprobebeuteln oder direkt mit einer nicht dargestellten Abgasmessanlage verbunden.

Zur Befüllung der Abgasprobenbeutel oder zur Herstellung der Verbindung zur Abgasmessanlage sind an der Decke 20 oder an den Seitenwänden 14, 16 des Schrankkörpers 12 nicht dargestellte Öffnungen ausgebildet, in denen entweder entsprechende Kupplungen zum Anschluss der Leitungen von außen ausgebildet sind oder in die die Leitungen direkt ins Innere des Schrankkörpers 12 führen. In gleicher Weise erfolgt auch ein elektrischer Anschluss der Messgeräte 24, 26, 28 an eine Stromquelle. Im Innern des Schrankkörpers 12 befinden sich entsprechend auch elektrische Leitungen zur Herstellung dieser Verbindung und zur Herstellung einer elektrischen Verbindung zu einer Bedieneinheit 34, welche als Touchscreen-Monitor 35 ausgeführt ist und in einer Öffnung 36 einer Tür 38 befestigt ist, welche über Scharniere 40 an der ersten Seitenwand 14 des Schrankkörpers 12 drehbeweglich befestigt ist, so dass eine offene Frontseite 42 des ersten Schrankkörpers 12 geöffnet und geschlossen werden kann. Über diese Bedieneinheit 34 kann sowohl die Abgasmessanlage als auch die einzelnen Messgeräte 24, 26, 28 bedient werden und deren Messergebnisse ausgelesen werden.

Ein baugleicher, zweiter Schrankkörper 44 ist erfindungsgemäß im geschlossenen Zustand hinter dem ersten Schrankkörper 12 angeordnet und ist über Gelenke 46 in Form von Scharnieren, die an der ersten Seitenwand 14 des ersten Schrankkörpers 12 und an einer ersten Seitenwand 48 des zweiten Schrankkörpers 44, befestigt sind, mit dem ersten Schrankkörper 12 verbunden. Dieser zweite Schrankkörper 44 weist entsprechend keine Tür auf, sondern dessen Frontseite 50 wird durch Drehung des ersten Schrankkörpers 12 um die Scharniere 46 freigegeben. Um diese Drehmöglichkeit und andererseits eine feste Positionierung des zweiten Schrankkörpers 44 sicher zu stellen, sind am Boden 18 des ersten Schrankkörpers 12 Rollen 52 befestigt. Ein Verschluss des zweiten Schrankkörpers 44 erfolgt über korrespondierende Verschlussmittel, welche an der zur ersten Seitenwand 14 gegenüberliegenden zweiten Seitenwand 16 des ersten Schrankkörpers 12 und einer zweiten Seitenwand 56 des zweiten Schrankkörpers 44 angeordnet sind. Nach dem Öffnen dieser Verschlussmittel kann der erste Schrankkörper 12 entsprechend zum zweiten Schrankkörper 44 um 180° gedreht werden, so dass zweite Messgeräte 58, 60, 62 im zweiten Schrankkörper 44 ebenfalls voll zugänglich sind.

Dieser zweite Schrankkörper 44 ist nicht nur baugleich mit dem ersten Schrankkörper 12, also mit den zwei Seitenwänden 48, 56, einer Decke 64, einem Boden 66 und einer Rückwand 68 ausgeführt, sondern ist auch in gleicher Weise ausgestattet mit einem Flammenionisationsdetektoranalysegerät 58, einem Chemilumineszenzdetektoranalysegerät 60 sowie einem Infrarotdetektoranalysegerät 62 sowie Gasanschlussleitungen, welche jedoch mit einer parallel geschalteten zweiten Abgasmessanlage fluidisch verbunden sind. Elektrische Leitungen, über die die Messgeräte 58, 60, 62 oder die zweite Abgasmessanlage vom Bedienpersonal gesteuert werden, sind jedoch direkt zur Tür 38 und der dort angeordneten Bedieneinheit 34 am ersten Schrankkörper 12 geführt.

Der erfindungsgemäße Schaltschrank eignet sich entsprechend, um zwei Abgasmessanlagen simultan über einen einzelnen Bediener zu steuern. Der hierzu benötigte Bauraum wird minimiert, indem lediglich eine Bedieneinheit für zwei Abgasmessanlagen verwendet werden muss. Dennoch bleibt eine vollständige Zugänglichkeit aller Messgeräte im Schaltschrank erhalten. Auch sind alle Aggregate der beiden Messanlagen räumlich voneinander getrennt und können selbstverständlich auch einzeln betrieben werden. Das Öffnen beider Schrankkörper ist durch einfaches Aufklappen möglich.

Es sollte deutlich sein, dass der Schutzbereich des vorliegenden Hauptanspruchs nicht auf das beschriebene Ausführungsbeispiel begrenzt ist. Insbesondere können die Schränke auch mit anderen oder zusätzlichen Messgeräten wie FTIR-Analysegeräten oder Quantenkaskadenlasern, CVS-Baugruppen, Analysebeuteln oder Partikelsammelsystemen bestückt werden. Auch die Anordnung sowie die Verbindungen der Bauteile zueinander und zur jeweiligen Abgasmessanlage können geändert werden. Ebenso ist es möglich, unterschiedliche Messgeräte für nur eine Abgasmessanlage in den beiden Schrankkörpern unterzubringen und durch den erfindungsgemäßen Schaltschrank deren Zugänglichkeit bei minimalem Platzbedarf zu bewahren.

## Patentansprüche

1. Schaltschrank (10) für Abgasmessanlagen mit
einem ersten Schrankkörper (12), in dem erste Messgeräte (24, 26, 28) zur Analyse von Probengasen angeordnet sind,
einer Tür (38) zum Öffnen und Schließen einer offenen Frontseite (42) des ersten Schrankkörpers (12), die drehbar am ersten Schrankkörper (12) befestigt ist und
einer Bedieneinheit (34), die an der Tür (38) angeordnet ist,
**dadurch gekennzeichnet, dass**
der erste Schrankkörper (12) relativ zu einem zweiten Schrankkörper (44) geführt bewegbar ist, in dem zweite Messgeräte (58, 60, 62) angeordnet sind und der eine zweite offene Frontseite (50) aufweist, welche durch die geführte Relativbewegung des ersten Schrankkörpers (12) zum zweiten Schrankkörper (44) verschließbar und freigebbar ist.

2. Schaltschrank für Abgasmessanlagen nach Anspruch 1,
**dadurch gekennzeichnet, dass**
der zweite Schrankkörper (44) über ein Gelenk (46) am ersten Schrankkörper (12) befestigt ist und der erste Schrankkörper (12) zum zweiten Schrankkörper (44) um das Gelenk (46) drehbar angeordnet ist.

3. Schaltschrank für Abgasmessanlagen nach einem der Ansprüche 1 oder 2,
**dadurch gekennzeichnet, dass**
die Messgeräte (58, 60, 62) im zweiten Schrankkörper (44) elektrisch mit der Bedieneinheit (34) an der Tür (38) des ersten Schrankkörpers (12) verbunden sind.

4. Schaltschrank für Abgasmessanlagen nach einem der vorhergehenden Ansprüche,
**dadurch gekennzeichnet, dass**
die Messgeräte (24, 26, 28) im ersten Schrankkörper (12) einer ersten Abgasmessanlage zugeordnet sind und die Messgeräte (58, 60, 62) im zweiten Schrankkörper (44) einer zweiten Abgasmessanlage zugeordnet sind.

5. Schaltschrank für Abgasmessanlagen nach einem der vorhergehenden Ansprüche,
**dadurch gekennzeichnet, dass**
an einem Boden (18) des ersten Schrankkörpers (12) Rollen (52) angeordnet sind.

6. Schaltschrank für Abgasmessanlagen nach einem der vorhergehenden Ansprüche,
**dadurch gekennzeichnet, dass**
der erste Schrankkörper (12) mit seiner Rückwand (22) gegen die offene Frontseite (50) des zweiten Schrankkörpers (44) anliegt.

7. Schaltschrank für Abgasmessanlagen nach einem der vorhergehenden Ansprüche,
**dadurch gekennzeichnet, dass**
der erste Schrankkörper (12) und der zweite Schrankkörper (44) sowie die im ersten Schrankkörper (12) und im zweiten Schrankkörper (44) angeordneten Messgeräte (24, 26, 28; 58, 60, 62) gleich sind.

8. Schaltschrank für Abgasmessanlagen nach einem der vorhergehenden Ansprüche,
**dadurch gekennzeichnet, dass**
der erste Schrankkörper (12) über mehrere an einer ersten Seitenwand (14) angeordnete, als Gelenk (46) dienende Scharniere an einer ersten Seitenwand (48) des zweiten Schrankkörpers (44) drehbar befestigt ist und an den jeweils gegenüberliegenden zweiten Seitenwänden (16, 56) der beiden Schrankkörper (12, 44) Verschlussmittel (54) angeordnet sind, über die die zweite Seitenwand (16) des ersten Schrankkörpers (12) mit der zweiten Seitenwand (56) des zweiten Schrankkörpers (44) verbindbar ist.

9. Schaltschrank für Abgasmessanlagen nach einem der vorhergehenden Ansprüche,
**dadurch gekennzeichnet, dass**
die Bedieneinheit (34) einen Monitor (35) aufweist, der in einer Öffnung (36) der Tür (38) angeordnet ist.

## Claims

1. A switchgear cabinet (10) for exhaust gas measuring facilities, comprising a first cabinet body (12) in which first measuring appliances (24, 26, 28) for the analysis of sample gases are arranged,
a door (38) for opening and closing an open front side (42) of said first cabinet body (12), which is rotatably attached to said first cabinet body (12), and
an operating unit (34) arranged on said door (38),
**characterized in that**
said first cabinet body (12) can be moved in a guided manner in relation to a second cabinet body (44) containing second measuring appliances (58, 60, 62) and comprising a second front side (50) that can be closed and opened by means of the guided relative movement of said first cabinet body (12) in relation to said second cabinet body (44).

2. The switchgear cabinet for exhaust gas measuring facilities according to claim 1,
**characterized in that**
the second cabinet body (44) is attached to the first cabinet body (12) via an articulated connection (46) and said first cabinet body (12) is arranged such that it is adapted to be rotated about said articulated connection (46) in relation to said second cabinet body (44).

3. The switchgear cabinet for exhaust gas measuring facilities according to any one of claims 1 or 2,
**characterized in that**
the measuring appliances (58, 60, 62) in the second cabinet body (44) are electrically connected to the operating unit (34) at the door (38) of the first cabinet body (12).

4. The switchgear cabinet for exhaust gas measuring facilities according to any one of the preceding claims,
**characterized in that**
the measuring appliances (24, 26, 28) in the first cabinet body (12) are assigned to a first exhaust gas measuring facility and the measuring appliances (58, 60, 62) in the second cabinet body (44) are assigned to a second exhaust gas measuring facility.

5. The switchgear cabinet for exhaust gas measuring facilities according to any one of the preceding claims,
**characterized in that**
at a bottom (18) of the first cabinet body (12) rollers (52) are arranged.

6. The switchgear cabinet for exhaust gas measuring facilities according to any one of the preceding claims,
**characterized in that**
the rear wall (22) of the first cabinet body (12) rests against the open front side (50) of the second cabinet body (44).

7. The switchgear cabinet for exhaust gas measuring facilities according to any one of the preceding claims,
**characterized in that**
the first cabinet body (12) and the second cabinet body (44) as well as the measuring appliances (24, 26, 28; 58, 60, 62) arranged in said first cabinet body (12) and said second cabinet body (44) are the same.

8. The switchgear cabinet for exhaust gas measuring facilities according to any one of the preceding claims,
**characterized in that**
the first cabinet body (12) is rotatably attached to a first side wall (48) of the second cabinet body (44) via a plurality of hinges arranged at a first side wall (14) and serving as an articulated connection (46) and closing means (54) are arranged at the respective opposite side walls (16, 56) of said two cabinet bodies (12, 44), via which said second side wall (16) of said first cabinet body (12) is adapted to be connected to said second side wall (56) of said second cabinet body (44).

9. The switchgear cabinet for exhaust gas measuring facilities according to any one of the preceding claims,
**characterized in that**
the operating unit (34) includes a monitor (35) which is arranged in an opening (36) of the door (38).

## Revendications

1. Armoire de commande (10) pour systèmes de mesure de gaz d'échappement comprenant
un premier corps d'armoire (12) dans lequel des premiers dispositifs de mesure (24, 26, 28) sont disposés pour analyser des gaz de prélèvement, une porte (38) pour ouvrir et fermer une face avant (42) ouverte du premier corps d'armoire (12), ladite face étant fixée de manière rotative au premier corps d'armoire (12) et
une unité de commande (34) disposée sur la porte (38),
**caractérisé en ce que**
le premier corps d'armoire (12) est déplaçable de manière guidée par rapport à un deuxième corps d'armoire (44), dans lequel deuxièmes dispositifs de mesure (58, 60, 62) sont disposés et qui présente une deuxième face avant (50) ouverte qui peut être fermée ou libérée par le mouvement guidé du premier corps d'armoire (12) par rapport au deuxième corps d'armoire (44).

2. Armoire de commande pour systèmes de mesure de gaz d'échappement selon la revendication 1, **caractérisé en ce que** le deuxième corps d'armoire (44) est monté au premier corps d'armoire (12) via une charnière (46) et le premier corps d'armoire (12) est disposé pour tourner autour de la charnière (46) par rapport au deuxième corps d'armoire (44).

3. Armoire de commande pour systèmes de mesure de gaz d'échappement selon l'une des revendications 1 ou 2, **caractérisé en ce que** les dispositifs de mesure (58, 60, 62) dans le deuxième corps d'armoire (44) sont connectés électriquement à l'unité de commande (34) sur la porte (38) du premier corps d'armoire (12).

4. Armoire de commande pour systèmes de mesure de gaz d'échappement selon l'une quelconque des revendications précédentes, **caractérisé en ce que** les dispositifs de mesure (24, 26, 28) dans le premier corps d'armoire (12) sont associés à un premier système de mesure de gaz d'échappement et les dispositifs de mesure (58, 60, 62) dans le deuxième corps d'armoire (44) sont associés à un deuxième système de mesure des gaz d'échappement.

5. Armoire de commande pour systèmes de mesure de gaz d'échappement selon l'une quelconque des revendications précédentes, **caractérisé en ce que** des rouleaux (52) sont disposés sur un plancher (18) du premier corps d'armoire (12).

6. Armoire de commande pour systèmes de mesure de gaz d'échappement selon l'une quelconque des revendications précédentes, **caractérisé en ce que** le premier corps d'armoire (12) s'appuie avec sa paroi arrière (22) sur la face avant (50) ouverte du deuxième corps d'armoire (44).

7. Armoire de commande pour systèmes de mesure de gaz d'échappement selon l'une quelconque des revendications précédentes, **caractérisé en ce que** le premier corps d'armoire (12) et le deuxième corps d'armoire (44) ainsi que les dispositifs de mesure (24, 26, 28; 58, 60, 62) disposés dans le premier corps d'armoire (12) et dans le deuxième corps d'armoire (44) sont identiques.

8. Armoire de commande pour systèmes de mesure de gaz d'échappement selon l'une quelconque des revendications précédentes, **caractérisé en ce que** le premier corps d'armoire (12) est monté de manière rotative sur une première paroi latérale (48) du deuxième corps d'armoire (44) par plusieurs charnières disposées sur une première paroi latérale (14) et servant de joint (46), et **en ce que** des moyens de fermeture (54) sont disposés sur deuxièmes parois latérales opposées (16, 56) des deux corps d'armoire (12, 44), par l'intermédiaire desquels la deuxième paroi latérale (16) du premier corps d'armoire (12) peut être relié à la seconde paroi latérale (56) du deuxième corps d'armoire (44).

9. Armoire de commande pour systèmes de mesure de gaz d'échappement selon l'une quelconque des revendications précédentes, **caractérisé en ce que** l'unité de commande (34) comporte un moniteur (35) disposé dans une ouverture (36) de la porte (38).
